# EUROPEAN PATENT SPECIFICATION

(11) **EP 4 257 045 B1**
(45) Date of publication and mention of the grant of the patent: **11.12.2024**
(21) Application number: 22166952.6
(22) Date of filing: 06.04.2022
(51) Int. Cl.: A61B 5/24, A61B 5/00, A61N 1/05

(54) **BASICALLY WIRE-SHAPED ELECTRODE FOR TRANSMITTANCE OF ELECTROPHYSIOLOGICAL NEUROSIGNALS AND METHOD OF MANUFACTURE THEREOF**
IM WESENTLICHEN DRAHTFÖRMIGE ELEKTRODE ZUR ÜBERTRAGUNG ELEKTROPHYSIOLOGISCHER NEUROSIGNALE UND VERFAHREN ZU IHRER HERSTELLUNG
ÉLECTRODE ESSENTIELLEMENT EN FORME DE FIL POUR LA TRANSMISSION DE NEUROSIGNAUX ÉLECTROPHYSIOLOGIQUES ET SON PROCÉDÉ DE FABRICATION

(43) Date of publication of application: 11.10.2023
(73) Proprietor: Leibniz-Institut für Neurobiologie, 39118 Magdeburg (DE); Otto-von-Guericke-Universität Magdeburg, 39106 Magdeburg (DE)
(72) Inventor: TAKAGAKI, Kentaroh, 39104 Magdeburg (DE); HERRERA-MOLINA, Rodrigo, 39104 Magdeburg (DE); ECKE, Martin, 32112 Magdeburg (DE); WILKE, Markus, 39110 Magdeburg (DE); WEBER, André, 39106 Magdeburg (DE); XIA, Zifeng, 39104 Magdeburg (DE)
(74) Representative: Günther, Constantin

(56) References cited:
- WO-A1-2021/009250
- JP-A- 2012 232 181
- US-A1- 2020 056 299
- US-A1- 2021 369 168
- US-A1- 2021 370 053
- US-B2- 10 426 362

## Description

### Technical Field

In a first aspect, the present invention relates to an improved basically wire-shaped electrode for transmittance of electrophysiological neurosignals as defined in claim 1. In another aspect, the present invention relates to a method according to claim 8 to manufacture the basically wire-shaped electrode of claim 1.

### Prior art

Implantable neural interfaces comprising wire-shaped electrodes have the potential to significantly improve clinical therapy and research by recording electrical signals from cells and/or stimulating cells electrically *in vivo, ex vivo* tissue preparations and / or from organoids. A prominent example for an application of such an electrode is deep brain stimulation.

EP 3915462 A1 reveals an electronic device for recording electrophysiological neurosignals comprising a bundle of insulated electric cables made of electrically conductive material and an insulation layer. WO 2021/009250 A1 discloses an implant, ensemble comprising such an implant and a method for fabrication such an implant.

Commercially available equipment comprises for example parylene-C-encapsulated silicon-based Utah microelectrode arrays with iridium coatings, silicon-based neural probes fabricated with complementary metal-oxide-semiconductor microtechnology and microelectromechanical system.

Current challenges include loss of contact to neurons due to relative movement between the implanted electrode and the tissue or brain or other location of a connected or targeted cell. Another challenge is the loss of signal due to an inflammation reaction followed by encapsulation, isolation and/or rejection of the implanted electrode. Furthermore, current electrodes often connect to multiple neuronal cells whereas transmittance of signals from and/or to a single or only few cells is more precise and therefore desirable. An improved electrode for the transmittance of electrophysiological neurosignals is therefore needed.

### Description of the invention

The present invention comprises such an improved electrode. The electrode substrate is basically wire-shaped and comprises an electrically conductive core and an electrically non-conductive surface layer. The electrically non-conductive surface layer therefore insulates the electrically conductive core. The electrode can be connected to an electrical unit and/or micro-electrical device to measure and control electrical signals transmitted by or to the electrode. The electrode can be used to transmit electrical signals from and/ or to at least one electrically active cell like cardiac cells, muscle cells or nerve cells from a variaty of tissues *in vivo, ex vivo* and /or organoids. The electrically conductive core can comprise one continuous wire, or multiple wires. The multiple wires can be twisted or otherwise connected.

Basically wire-shaped substrate describes an elongated body, the electrically conductive core, which is completely enclosed by a hollow body, the electrically non-conductive layer, in one extension direction. In this extension direction, in a three-dimensional coordinate diagram, the substrate is at least twice as long as in the two directions perpendicular to it. The ends of the wire-shaped substrate are therefore the area which is defined by the two short axes and show the electrically conductive core surrounded by the electrically non-conductive surface layer. The ends of the basically wire-based substrate can be covered with electrically non-conductive surface layer material. The elongated body can be a cylinder or multiple coiled and/ or twisted cylinders. The electrically non-conductive surface layer material can have a uniform thickness and therefore follow the inner body shape or create a different visible substrate shape like a rounded rectangle or cylinder.

The electrically conductive core can be made of platinum-iridium-wire. The electrically non-conductive surface layer can be made of polytetrafluoroethylene (PTFE). These materials are very flexible and improve the biocompatibility with reduced inflammatory response. Of course, other conductive and non-conductive materials can be used as well.

The electrically non-conductive surface layer has at least one opening to the electrically conductive core material reaching into the electrically conductive core by removing electrically conductive core material. The therefore accessible contiguous electrically conductive surface area is the location where electrical signals can be transmitted from and/or to electrically active cells. The surface(s) where the at least one electrically active cell transmit and/or receive electrical signals is defined as the contact area(s).

This enables the transmission of electrical signals to and/or from at least one electrically active cell like for example neural tissue or neurons via the contact area(s) of electrically conductive material. The contact area is located at the opening in the electrically non-conductive surface layer. The contact area can be the electrically conductive core material or any other electrically conductive material electrically conductively connected to the electrically conductive core material with an accessible surface.

The at least one opening to the electrically conductive core is created by at least one hole in the electrically conductive core material to below the surface of the electrically conductive core, e.g. like a cavity or blind hole. The hole in the electrically conductive core material can be 1 µm deep or deeper, like 5 µm or more, like 10 µm or more. The contact area therefore has a surface area that is larger than the area that would have been accessible if only the electrically non-conductive surface layer would have been removed. The enlargement of the contact area improves the signal-to-noise ratio and facilitates the connection to at least one electrically active cell. Due to the improved signal-to-noise ratio even smaller contact areas are possible, where only a few or even a single electrically active cell can bind. Transmittance from and/or to only few or single electrically active cells massively improves the accuracy of measurement and/or therapy.

These advantages allow the basically wire-shaped electrode substrate to have a small maximum diameter of 60 µm or less, like 50 µm or less, or 35 µm or less, or 20 µm or less, or 10 µm or less. This small diameter reduces relative movement of the electrode and surrounding tissue which enables long-term studies without loss of connection to the bound at least one electrically active cell. The small electrode diameter also improves the flexibility of the electrode which can come in handy when implanting it into neural tissue and increases comfort when used *in vivo, ex vivo* and/ or in organoids.

A thinner electrode is also less invasive. Also, the surface enlargement achieved by removing electrically conductive core material, in contrast to building up a structure on top of the basically wire-shaped electrode beyond the original dimensions, is less invasive to the surrounding tissue. Therefore, the present electrode reduces the inflammation reaction and encapsulation of the electrode and prevents the electrode from being rejected. Consequently, it enables long-term measurement and/or therapy electrically active cells *in vivo, ex vivo* and/or from organoids.

In one embodiment, the electrode has at least one contact area that has at least one pillar-like structure extending away from the surface of the contact area. The at least one pillar-like structure can be based on the ground of the aforementioned hole in the electrically conductive core material. The at least one pillar-like structure can be higher than the hole created removing electrically conductive core material. The at least one pillar-like structure can exceed the original dimensions of the basically wire-shaped electrode substrate. The at least one pillar-like structure can be 1 µm high or more, like 5 µm or more, like 10 µm or more. The at least one pillar-like structure can be part of the contact area. The at least one pillar-like structure can cover the entire contact area or only a part of it.

The at least one pillar-like structure may be wholly or partially of electrically conductive material. The preferred material(s) is/are biocompatible material(s). Suitable biocompatible materials are known to the person skilled in the art. Examples for conductive biocompatible materials are gold, platinum and titanium. The at least one pillar-like structure may be conductively connected to the electrode to detect and/or transmit electrical signals. The diameter of the at least one pillar-like structure can be 10 µm or less. The at least one pillar-like structure may be cuboid, cylindrical, cone-shaped or tapering. The free end of the at least one pillar-like structure may be tapered. The free end of the at least one pillar-like structure may be rounded. The at least one pillar-like structure improves the affinity of the contact area to bind electrically active cells. The at least one pillar-like structure also increases the likelihood of binding electrically active cells.

In one embodiment, the at least one opening to the electrically conductive core reaching into the electrically conductive core also comprises the opening through the at least partially pre-coating. The at least partially pre-coating can be located on top of the electrode substrate, more precisely on the electrically non-conductive surface layer. The pre-coating material can be biocompatible. The pre-coating can have a height of 50 nm or less. Examples for suitable conductive biocompatible materials are gold, platinum and carbon. The pre-coating can improve the deposition of further material to the electrically non-conductive surface layer.

In another embodiment, at least one additional layer of electrically conductive material, called a protective bar, is located on top of the electrically non-conductive surface layer. The protective bar establishes some kind of wall or barrier around the at least one opening. The protective bar can have a height of 500 nm or less, or 400 nm or less, 300 nm or less, 200 nm or less, 100 nm or less. The protective bar can have a circular shape or any other shape like a rectangular shape or triangular shape. The protective bar can have for example a diameter of 50 µm when circular, or an edge length of 40 to 20 µm or any other size fitting on the electrode. Of course, any other dimensions are also possible so that the size of the protective bar can be adjusted to the desired shape of the at least one opening in the electrically non-conductive surface layer. In one embodiment, the protective bar is located on top of the at least partially pre-coating above the electrically non-conductive surface layer. The protective bar protects the basically wire-shaped electrode substrate against heat generation during the fabrication process.

In one embodiment, the at least one contact area is located in a way on the electrode substrate that the at least one opening in the electrically non-conductive surface layer is at least partially surrounded by either the at least partially pre-coating or the protective bar or both of them.

In one embodiment, the at least one contact area is at least partially coated, the coating named for this purpose the contact area coating. The contact area coating may be made of carbon, carbon nanotubes, or a metal, such as gold, silver, aluminum, chromium, titanium, platinum. The contact area coating may result in an increased contiguous conductive surface area and therefore improve the signal-to-noise ratio as well as the adhesion of the sample. The contact area coating can also protect the coated contact area from oxidation. The contact area coating can also comprise the coating of at least one pillar-like structure.

Due to the small diameter of the wire-based electrode substrate and the small at least one hole, only a few or even a single electrically active cell may connect to the contact area per one contact area. This drastically improves the information gained and therapeutic effect that may be achieved by the electrode.

In a second aspect, the invention relates to the manufacture of one of the basically wire-shaped electrode described above. The manufacture starts with providing or producing a basically wire-shaped electrode substrate. The basically wire-shaped electrode substrate comprises an electrically conductive core and an electrically non-conductive surface layer. The basically wire-shaped electrode substrate can have a maximum diameter of 60 µm or less, like 50 µm or less, or 35 µm or less, or 20 µm or less, or 10 µm or less. The material of the electrically conductive core is surrounded by an electrically non-conductive surface layer. The electrically conductive core material can be platinum-iridium or any other complementary conductive metal or semiconductors..

In the second step, at least one opening is made in the electrically non-conductive surface layer that extends into the material of the electrically conductive core and therefore creates a hole into the electrically conductive core. The at least one opening can be of any shape, for example circular, rectangular or triangular or any other shape. The created hole in the electrically conductive core can be of 1 µm to 10 µm depth or deeper. The hole in the electrically conductive core may have at least partially rounded boundary surfaces. The hole can also have at least partially flat and/or angled boundary surfaces. The at least one opening can be made by removal of material.

In one embodiment, prior to the manufacture of the at least one opening, a layer of conductive material can be deposited at least partially on the electrically non-conductive surface layer, for this purpose named the pre-coating. In one embodiment, the electrode substrate is at least partially pre-coated with an electrically conductive material prior to creating the at least one opening. The material of this deposited electrically conductive pre-coating can be platinum or any other biocompatible, conductive material like gold, chromium, titanium among others. The pre-coating can be deposited by physical vapor deposition or other techniques creating a thin film. The pre-coating can have a height of 50 nm or less. Examples for suitable conductive biocompatible materials are gold, platinum and carbon. The pre-coating can exceed the dimensions of the at least one opening in the electrically non-conductive surface layer in at least one direction.

In another embodiment, the manufacture of the electrode can comprise the deposition of at least one layer of at least one conductive material in at least one spot named the protective bar. The protective bar can be made of platinum, gold or any other metal or any other conductive material.

The protective bar can have a height of 500 nm or less, or 400 nm or less, 300 nm or less, 200 nm or less, 100 nm or less. The protective bar can have a circular shape or any other shape like a rectangular shape or triangular shape. Of course, any other dimensions are also possible so that the size of the protective bar can be adjusted to the desired shape of the at least one opening in the electrically non-conductive surface layer. The protective bar can have for example a diameter of 50 µm when circular, or an edge length of 40 to 20 µm or any other size fitting on the electrode. In one embodiment, the at least one protective bar is deposited directly on the electrically non-conductive surface layer of the electrode substrate. In another embodiment, the at least one protective bar is deposited on top of the at least partially pre-coating above the electrically non-conductive surface layer. The at least one protective bar can be manufactured by material deposition on the electrically non-conductive surface layer or the pre-coating by a focused ion beam. In one embodiment, the deposition can be executed by focused ion beam with platinum precursor gas at a current of the ion beam of less than approximately 1.0 nA. The at least one protective bar can be manufactured with a height of 500 nm or less, or 400 nm or less, 300 nm or less, 200 nm or less, 100 nm or less. The at least one protective bar can be manufactured having a circular shape or any other shape like a rectangular shape or triangular shape. The protective bar can have for example a diameter of 50 µm when circular, or an edge length of 40 to 20 µm or any other size fitting on the electrode. The side lengths of the at least one protective bar can be 60 µm or less, like 50 µm or less, or 35 µm or less, or 20 µm or less, or 10 µm or less. Those lengths that exceed the diameter of the basically wire-shaped electrode substrate can in one embodiment be understood as approximately aligned with the longitudinal axis of the basically wire-shaped electrode substrate. In one embodiment, the protective bar can be manufactured at least partially on top of the pre-coating. The protective bar can be of different material than the pre-coating described before or can be the same material. The electrode manufacture can comprise only the manufacture of the at least one opening and hole to and into the electrically conductive core.

The at least one opening can be made through the protective bar and/or the pre-coating. The at least one opening in the basically wire-shaped electrode substrate can be made at least partially penetrating the pre-coating and/or protective bar leaving the at least one opening at least partially surrounded by the pre-coating and/or protective bar material on top of the electrically non-conductive surface layer of the basically wire-shaped electrode substrate. The protective bar can protect the basically wire-shaped electrode substrate from being destroyed by heat generation during the further fabrication process including the fabrication of the at least one opening and corresponding the hole in the electrically conductive core material.

In another embodiment, the at least one opening in the electrically non-conductive surface layer and/or the hole in the electrically conductive core can be made at least partially by a focused ion beam. Processes using focused ion beam to remove material are called herein subtractive processes. Processes using focused ion beam, potentially in combination with necessary other substances like precursor gas, to deposit material are called additive processes. The at least one protective bar can be manufactured by an additive process. The at least one opening can be manufactured by a subtractive process.

Pre-coating can take place prior to the manufacture of the at least one opening in the electrically non-conductive surface layer or prior to the manufacture of at least one protective bar. The pre-coating can be a thin film. The pre-coating can improve the deposition of conductive material on the surface of the electrically non-conductive substrate surface layer by additive processes, for example the subsequent manufacture of at least one protective bar on top of the pre-coating.

In one embodiment, the manufacture of the electrode comprises the manufacture of at least one pillar-like structure on the contact area created by a hole in the electrically conductive core. The pillar-like structure can be manufactured by additive and/or subtractive processes using a focused ion beam. The pillar-like structure can for example be manufactured by adding material on top of to the contact area and then sculpting it with a follow-up subtractive process. The pillar-like structure can be manufactured of electrically conductive material. During the manufacture of the at least one pillar-like structure additional electrically conductive core material can be removed.

A plurality of additive and/or subtractive processes can be strung together to manufacture an electrode of the type previously described. Subtractive processes can be executed at a current of the ion beam of approximately 1.0 nA and greater to remove material. Additive processes may for example be executed at a current of the ion beam of less than approximately 1.0 nA in combination with a suitable precursor gas to add material. Suitable precursor gases, settings and equipment for this purpose are known to those skilled in the art. However, the specification of the current is only a guide value and a clear differentiation of subtractive and additive processes by the current is not possible. Rather, the boundaries are blurred and depend on parameters such as residual gas pressure, area to be processed and others.

In another embodiment, the manufacture process can be supported by at least one microscopy imaging technique. With the microscopy imaging technique, the manufacture process can be controlled more precisely. For example, focused ion beam processes can be combined with a microscopy imaging technique. The microscopy imaging technique can be a Scanning electron microscopy (SEM) or any other high resolution microscopy imaging technique. This allows the focused ion beam to be aligned before and/or during fabrication. Advantageously, the microscopic method can also be used to determine when the subtractive and/or additive process can be terminated.

One electrode can comprise multiple contact areas that can be all uniform or have different sizes, shapes, depths, have, have partially or have not a pre-coating, have, have partially or have not a protective bar and/or have, have partially or have not at least one pillar-like structure. The distance between contact areas and every other parameter of the electrode described here, like the depth of the hole and the contact area coating material, can be adjusted to the individual patient and/or tissue. The electrode can be therefore multimodal and/or individualized. The electrode can be mounted for at least one of the manufacturing steps to a tiltable table. The article a/an is to be understood as an indefinite article in this application.

### Description of figures

- 101: electrically non-conductive surface layer
- 102: electrically conductive core
- 103: contact area
- 204: pre-coating
- 305: protective bar
- 606: contact area coating
- 707: pillar-like structure

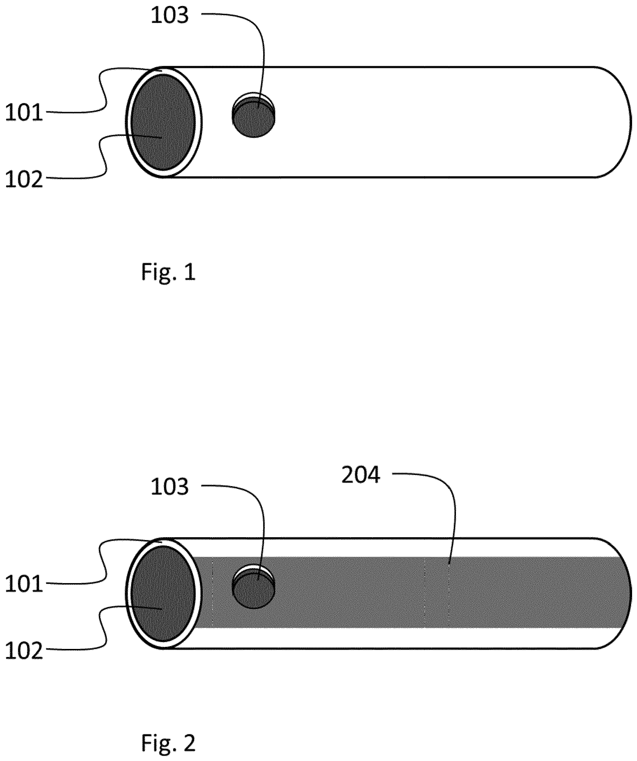

Figure 1 shows a basically wire-based electrode comprising an electrically non-conductive surface layer 101 and an electrically conductive core 102. The electrically non-conductive surface layer 101 has at least one opening to the electrically conductive core 102 material reaching into the electrically conductive core by removing electrically conductive core material. The contiguous area of conductive material is the contact area 103. The opening can be of any size or shape. The opening can be for example 10 µm deep into the electrically conductive core material 102.

Figure 2 shows a basically wire-based electrode comprising an electrically non-conductive surface layer 101, an electrically conductive core 102 and a partial pre-coating 204 on top of the electrically non-conductive surface layer 101. The electrode has an opening through the pre-coating, the electrically non-conductive surface layer 101 reaching into the electrically conductive core 102 material and creates the contact area 103.

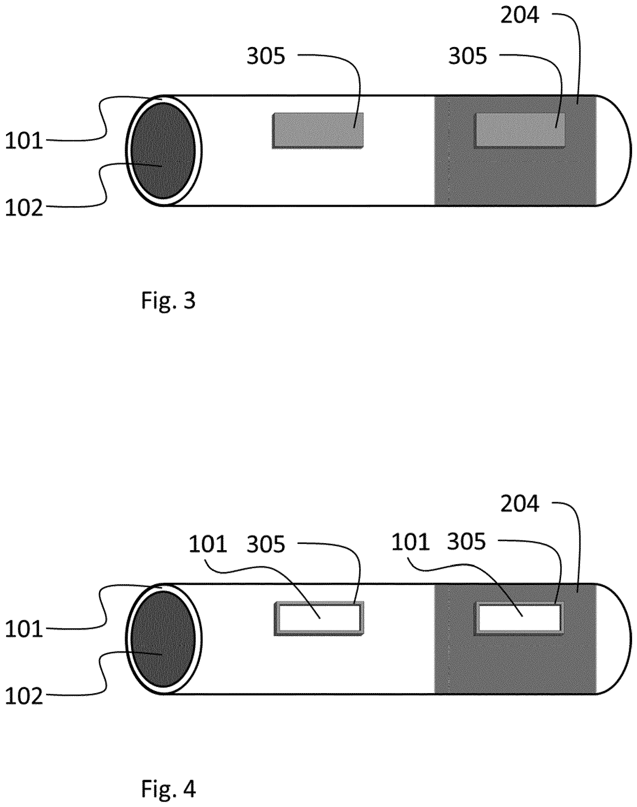

Figure 3 shows a manufacturing step of a basically wire-based electrode comprising an electrically non-conductive surface layer 101, an electrically conductive core 102 and a protective bar 305. The protective bar 305 can be manufactured by a focused ion beam. The electrode may as well have an at least partial pre-coating on top of the electrically non-conductive surface layer.

Figure 4 shows a manufacturing step of a basically wire-based electrode comprising an electrically non-conductive surface layer 101, an electrically conductive core 102 and a protective bar 305. The protective bar 305 has the shape of a frame. The protective bar can have any other shape. The empty center can be achieved by manufacture directly a frame or by removing material of a protective bar. The protective bar can be manufactured by a focused ion beam. The focused ion beam can also be used to remove the material of the protective bar. The electrode may as well have an at least partial pre-coating 204 between the electrically non-conductive surface layer 101 and the protective bar 305.

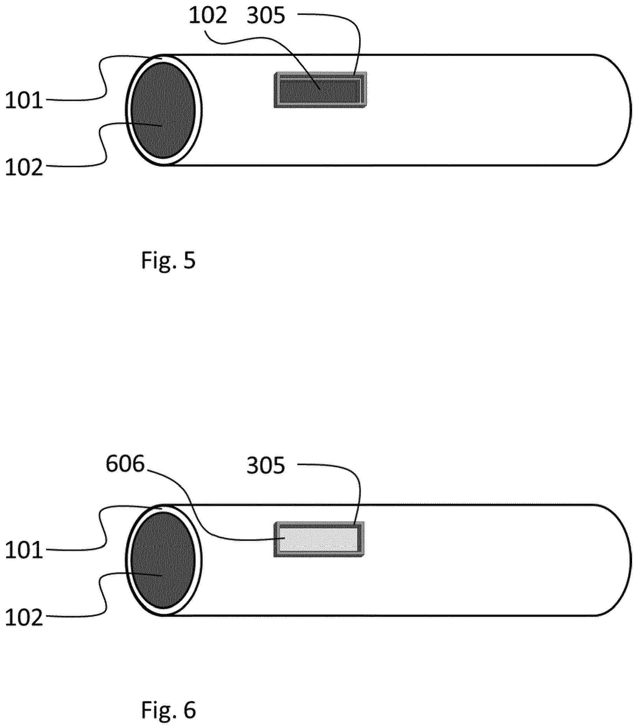

Figure 5 shows a basically wire-based electrode comprising an electrically non-conductive surface layer 101, an electrically conductive core 102 and a full frame of a protective bar 305. The electrode has an opening through the protective bar 305, the electrically non-conductive surface layer 101 reaching into the electrically conductive core 102 material. In another embodiment, a pre-coating can be placed between the electrically non-conductive surface layer and the protective bar 305.

Figure 6 shows a basically wire-based electrode comprising an electrically non-conductive surface layer 101, an electrically conductive core 102 and a full frame of a protective bar 305. The electrode has an opening through the protective bar 305, the electrically non-conductive surface layer 101 reaching into the electrically conductive core 102 material. The contact area is coated with the contact area coating 606. The contact area can be coated to enlarge the surface.

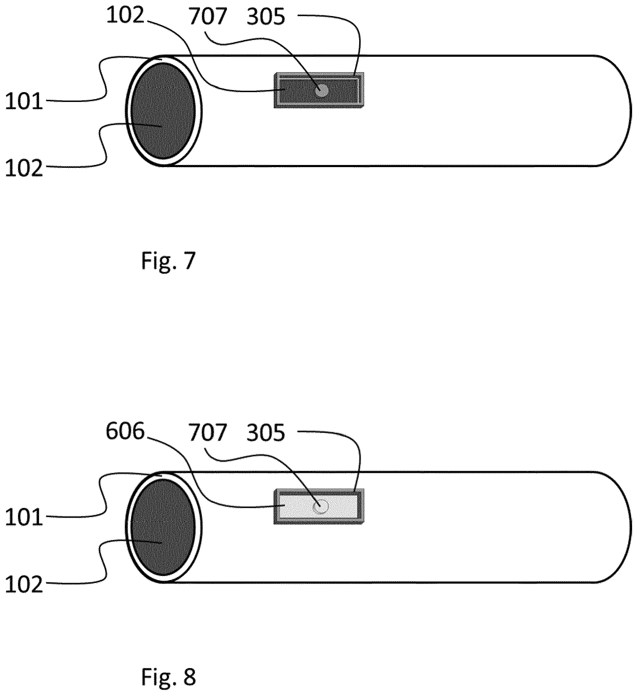

Figure 7 shows a basically wire-based electrode comprising an electrically non-conductive surface layer 101, an electrically conductive core 102 and a full frame of a protective bar 305. The electrode has an opening through the protective bar 305, the electrically non-conductive surface layer 101 reaching into the electrically conductive core 102 material. The contact area comprises a pillar-like structure 707. The pillar-like structure can be of any shape or size, for example the pillar-like structure can be 10 µm high and have a tapered shape. The pillar-like structure 707 can be manufactured using a focused ion beam.

Figure 8 shows a basically wire-based electrode comprising an electrically non-conductive surface layer 101, an electrically conductive core 102 and a full frame of a protective bar 305. The electrode has an opening through the protective bar 305, the electrically non-conductive surface layer 101 reaching into the electrically conductive core 102 material. The contact area comprises a pillar-like structure 707. The pillar-like structure can be of any shape or size, for example the pillar-like structure can be 10 µm high and have a tapered shape. The pillar-like structure 707 can be manufactured using a focused ion beam. The contact area is coated with the contact area coating 606. The contact area also comprises the coated pillar-like structure 707.

## Claims

1. A basically wire-based electrode for transmittance of electrophysiological neurosignals comprising a basically wire-shaped substrate with an electrically conductive core (102) and an electrically non-conductive surface layer (101), the electrically non-conductive surface layer (101) having at least one opening to the electrically conductive core (102) and a contact area (103) of electrically conductive material for the transmission of electrical signals to and/or from neural tissue, the contact area (103) being located at the opening in the insulation layer and electrically conductively connected to the substrate core wherein the at least one contact area (103) is formed by at least one hole in the substrate core material to below the surface of the electrically conductive core (102).

2. The electrode according to claim 1 wherein the wire-based substrate has a maximum diameter of 60 µm or less, like 50 µm or less, or 35 µm or less, or 20 µm or less, or 10 µm.

3. The electrode according to one of the claims above wherein the at least one contact area (103) has at least one pillar-like structure (707).

4. The electrode according to claim 3 wherein the at least one pillar-like structure (707) is cuboid, cylindrical, cone-shaped or tapering.

5. The electrode according to one of the claims above wherein the electrically non-conductive surface layer (101) is at least partially pre-coated with an electrically conductive material.

6. The electrode according to one of the claims above wherein the at least one opening is at least partially surrounded by a protective bar (305).

7. The electrode according to one of the claims above wherein at least part of the at least one contact area (103) is coated with a contact area coating (606).

8. A method for the manufacture of a wire-based electrode according to any of the preceding claims, the method comprising local removal of material including the electrically non-conductive surface layer (101) material and electrically conductive core (102) material.

9. A method according to claim 8, the method comprising the at least partial deposition of a pre-coating (204) on the electrically non-conductive surface layer (101).

10. A method according to one of the claims 9 to 10, comprising the deposition of at least one protective bar (305).

11. A method according to one of the claims 8 to 10, the method comprising the manufacture of at least one pillar-like structure (707) by deposition of material on and/or removal of material of the contact area (103).

12. A method according to one of the claims 8 to 11, the method comprising coating of at least part of the contact area (103) with a contact area coating (606).

13. A method according to one of the claims 8 to 12, wherein a focused ion beam is used for the deposition and/or removal of material in at least one manufacturing step.

14. A method according to one of the claims 8 to 13 wherein the deposition and/or removal of material is at least partially monitored with a microscopy imaging technique.

## Patentansprüche

1. Im Wesentlichen drahtbasierte Elektrode zur Übertragung elektrophysiologischer Nervensignale, umfassend ein im Wesentlichen drahtförmiges Substrat mit einem elektrisch leitenden Kern (102) und einer elektrisch nicht leitenden Oberflächenschicht (101), wobei die elektrisch nicht leitende Oberflächenschicht (101) mindestens eine Öffnung zum elektrisch leitenden Kern (102) und eine Kontaktfläche (103) aus elektrisch leitfähigem Material zur Übertragung elektrischer Signale zu und/oder von Nervengewebe aufweist, wobei die Kontaktfläche (103) an der Öffnung in der Isolationsschicht angeordnet und elektrisch leitend mit dem Substratkern verbunden ist, wobei die mindestens eine Kontaktfläche (103) durch mindestens ein Loch im Substratkernmaterial bis unter die Oberfläche des elektrisch leitenden Kerns (102) gebildet wird.

2. Elektrode nach Anspruch 1, wobei das drahtförmige Substrat einen maximalen Durchmesser von 60 µm oder weniger, wie 50 µm oder weniger, oder 35 µm oder weniger, oder 20 µm oder weniger, oder 10 µm aufweist.

3. Elektrode nach einem der vorstehenden Ansprüche, wobei die mindestens eine Kontaktfläche (103) mindestens eine säulenartige Struktur (707) aufweist.

4. Elektrode nach Anspruch 3, wobei die mindestens eine säulenartige Struktur (707) quaderförmig, zylindrisch, kegelförmig oder spitz zulaufend ist.

5. Elektrode nach einem der vorstehenden Ansprüche, wobei die elektrisch nicht leitende Oberflächenschicht (101) zumindest teilweise mit einem elektrisch leitenden Material vorbeschichtet ist.

6. Elektrode nach einem der vorstehenden Ansprüche, wobei die mindestens eine Öffnung zumindest teilweise von einem Schutzstab (305) umgeben ist.

7. Elektrode nach einem der vorstehenden Ansprüche, wobei mindestens ein Teil der mindestens einen Kontaktfläche (103) mit einer Kontaktflächenbeschichtung (606) beschichtet ist.

8. Verfahren zur Herstellung einer drahtbasierten Elektrode gemäß einem der vorstehenden Ansprüche, wobei das Verfahren das lokale Entfernen von Material umfasst, einschließlich des Materials der elektrisch nicht leitenden Oberflächenschicht (101) und des Materials des elektrisch leitenden Kerns (102).

9. Verfahren nach Anspruch 8, wobei das Verfahren die zumindest teilweise Abscheidung einer Vorbeschichtung (204) auf der elektrisch nicht leitenden Oberflächenschicht (101) umfasst.

10. Verfahren nach einem der Ansprüche 9 bis 10, umfassend das Aufbringen von mindestens einem Schutzstab (305).

11. Verfahren nach einem der Ansprüche 8 bis 10, wobei das Verfahren die Herstellung mindestens einer säulenartigen Struktur (707) durch Abscheidung von Material auf und/oder Entfernung von Material von der Kontaktfläche (103) umfasst.

12. Verfahren nach einem der Ansprüche 8 bis 11, wobei das Verfahren das Beschichten von mindestens einem Teil der Kontaktfläche (103) mit einer Kontaktflächenbeschichtung (606) umfasst.

13. Verfahren nach einem der Ansprüche 8 bis 12, wobei ein fokussierter lonenstrahl für die Abscheidung und/oder Entfernung von Material in mindestens einem Herstellungsschritt verwendet wird.

14. Verfahren nach einem der Ansprüche 8 bis 13, wobei die Abscheidung und/oder Entfernung von Material zumindest teilweise mit einer Mikroskopie-Bildgebungsmethode überwacht wird.

## Revendications

1. Électrode essentiellement à base filaire pour une transmittance de neurosignaux électrophysiologiques comprenant un substrat essentiellement de forme filaire avec un noyau électriquement conducteur (102) et une couche de surface électriquement non conductrice (101), la couche de surface électriquement non conductrice (101) ayant au moins une ouverture vers le noyau électriquement conducteur (102) et une zone de contact (103) en matériau électriquement conducteur pour la transmission de signaux électriques vers et/ou depuis un tissu neural, la zone de contact (103) étant située au niveau de l'ouverture dans la couche d'isolation et étant connectée de manière électriquement conductrice au noyau de substrat, dans laquelle ladite au moins une zone de contact (103) est formée par au moins un trou dans le matériau de noyau de substrat jusqu'à en dessous de la surface du noyau électriquement conducteur (102).

2. Électrode selon la revendication 1, dans laquelle le substrat à base filaire a un diamètre maximum de 60 µm ou moins, comme 50 µm ou moins, ou 35 µm ou moins, ou 20 µm ou moins, ou 10 µm.

3. Électrode selon l'une des revendications ci-dessus, dans laquelle ladite au moins une zone de contact (103) a au moins une structure de type pilier (707).

4. Électrode selon la revendication 3, dans laquelle ladite au moins une structure de type pilier (707) est cuboïde, cylindrique, en forme de cône ou effilée.

5. Électrode selon l'une des revendications ci-dessus, dans laquelle la couche de surface électriquement non conductrice (101) est au moins en partie pré-revêtue avec un matériau électriquement conducteur.

6. Électrode selon l'une des revendications ci-dessus, dans laquelle ladite au moins une ouverture est au moins en partie entourée par une barre protectrice (305).

7. Électrode selon l'une des revendications ci-dessus, dans laquelle une partie au moins de ladite au moins une zone de contact (103) est revêtue avec un revêtement de zone de contact (606).

8. Procédé de fabrication d'une électrode à base filaire selon l'une quelconque des revendications précédentes, le procédé un enlèvement local d'un matériau incluant le matériau de couche de surface électriquement non conductrice (101) et le matériau de noyau électriquement conducteur (102).

9. Procédé selon la revendication 8, le procédé comprenant le dépôt au moins partiel d'un pré-revêtement (204) sur la couche de surface électriquement non conductrice (101).

10. Procédé selon l'une des revendications 9 à 10, comprenant le dépôt d'au moins une barre protectrice (305).

11. Procédé selon l'une des revendications 8 à 10, le procédé comprenant la fabrication d'au moins une structure de type pilier (707) par dépôt et/ou enlèvement de matériau sur/de la zone de contact (103).

12. Procédé selon l'une des revendications 8 à 11, le procédé comprenant un revêtement d'une partie au moins de la zone de contact (103) avec un revêtement de zone de contact (606).

13. Procédé selon l'une des revendications 8 à 12, dans lequel un faisceau d'ions focalisés est utilisé pour le dépôt et/ou l'enlèvement de matériau dans au moins une étape de fabrication.

14. Procédé selon l'une des revendications 8 à 13, dans lequel le dépôt et/ou l'enlèvement de matériau est au moins en partie surveillé avec une technique d'imagerie par microscopie.
